# EUROPÄISCHE PATENTSCHRIFT

(11) **EP 3 192 477 B1**
(45) Veröffentlichungstag und Bekanntmachung des Hinweises auf die Patenterteilung: **23.06.2021**
(21) Anmeldenummer: 17150942.5
(22) Anmeldetag: 11.01.2017
(51) Int. Cl.: A61F 7/00

(54) **MEDIZINISCHES TEMPERIERGERÄT**
MEDICAL TEMPERATURE CONDITIONING APPARATUS
APPAREIL MÉDICAL DE RÉGULATION DE TEMPÉRATURE

(30) Priorität: 14.01.2016 DE 102016000344
(43) Veröffentlichungstag der Anmeldung: 19.07.2017
(73) Patentinhaber: Hilotherm Holding AG, 6317 Oberwil bei Zug (CH)
(72) Erfinder: Janisch, Klaus, 88316 Isny (DE)
(74) Vertreter: Wallinger, Michael

(56) Entgegenhaltungen:
- EP-A1- 2 359 781
- WO-A1-97/36560
- US-B1- 7 959 657

## Beschreibung

Die Heilwirkung von Wärme- und Kältebehandlungen für den menschlichen und tierischen Körper ist schon seit langem bekannt. Während einfache Temperierungseinrichtungen, wie Eisbeutel oder Wärmeflaschen, als Hausmittel nach wie vor Verwendung finden, hat man in der professionellen medizinischen Therapie erkannt, dass sich der Heilerfolg solcher Maßnahmen bedeutend verbessert, wenn man den betroffenen Körperbereich mit einer bestimmten, meist konstanten, Temperatur versorgt. Eine solche therapeutische Maßnahme, die sehr erfolgreich bei der Behandlung von traumatisierten oder entzündeten Gelenken eingesetzt wird, ist z. B. eine gleichmäßige Temperierung auf 15° C.

Um diese Temperierung zu erreichen, wird auf die betroffenen Körperbereiche eine Temperier-Manschette aufgelegt, die eine Vielzahl von Strömungswegen für Wasser enthält. Diese Manschette wird dann von einem Temperiergerät der hier in Rede stehenden Art mit temperiertem Wasser versorgt, so dass die gewünschte Temperatur über die Behandlungszeit im Wesentlichen konstant gehalten bzw. an einen gewünschten Temperaturverlauf angepasst werden kann.

Die bekannten Temperiergeräte werden sowohl im klinischen Bereich als auch im privaten Bereich eingesetzt. Für die Privatanwendung erwirbt der Patient entweder ein solches Gerät selbst oder mietet es für die Zeitdauer der Therapie. Dokumente EP2359781 A1, WO9736560 A1 und US7959657 B1 offenbaren einschlägigen Stand der Technik.

Es zeigt sich nun, dass die Inbetriebnahme und die Handhabung der bekannten Geräte gerade, wenn diese im häuslichen Bereich eingesetzt werden, oft nicht so einfach und komfortabel ist, wie dies für den Privatanwender wünschenswert wäre.

Die vorliegende Erfindung stellt sich deshalb die Aufgabe, den Komfort bei der Benutzung eines solchen medizinischen Temperiergerätes zu erhöhen.

Diese Aufgabe wird erfindungsgemäß durch den Gegenstand des Anspruchs 1 gelöst. Zu bevorzugende Weiterbildungen der Erfindung sind Gegenstand der Unteransprüche.

Ein medizinisches Temperiergerät der vorliegend betrachteten Art zur Temperierung eines Fluids hat wenigstens ein Gehäuse, wenigstens eine Fluidversorgungseinrichtung, durch welche ein Fluid in dem Gehäuse bereitgestellt wird, wenigstens eine im Gehäuse angeordnete Temperiereinrichtung, die das durch das Gehäuse strömende Fluid temperiert, wenigstens eine Fluidabströmungseinrichtung, durch welche das Fluid mit einer im Wesentlichen vorbestimmten Temperatur aus dem Gehäuse herausgeführt wird, und wenigstens eine im Gehäuse angeordnete Pumpeneinrichtung, welche das durch das Gehäuse strömende Fluid mit Druck beaufschlagt.

Unter einer Fluidversorgungseinrichtung wird dabei eine Einrichtung verstanden, die das Vorhandensein des Fluids während des Betriebs des Temperiergerätes in stets ausreichender Menge sicherstellt oder es dem Benutzer des Temperiergerätes ermöglicht, dies zu tun, insbesondere durch geeignete Mittel zur Bevorratung und/oder Nachfüllung des Fluids.

Unter einer Temperiereinrichtung wird eine Einrichtung zum Heizen und/oder Kühlen des Fluids verstanden, vorzugsweise durch Umwandlung von elektrischer Energie in Wärmeenergie bzw. durch Verwendung von elektrischer Energie zum Entziehen von Wärmeenergie und vorzugsweise mit einem Wärmetauscher zur Übertragung von erzeugter Wärme auf das Fluid bzw. zum Entziehen von Wärme aus dem Fluid.

Erfindungsgemäß weist die wenigstens eine Temperiereinrichtung wenigstens eine erste Temperiereinheit und wenigstens eine zweite Temperiereinheit auf, welche jeweils wenigstens ein thermoelektrisches Element, insbesondere wenigstens ein Peltier-Element, wenigstens ein von Fluid durchströmbares Wärmetauscherelement zur Übertragung von Wärme auf das oder von dem Fluid und wenigstens einen Kühlkörper aufweisen. Dabei sind der wenigstens eine Kühlkörper der wenigstens einen ersten Temperiereinheit und der wenigstens eine Kühlkörper der wenigstens einen zweiten Temperiereinheit einander zugewandt.

Das Fluid ist vorzugsweise Wasser, insbesondere Leitungswasser, kann aber auch destilliertes Wasser, Wasser mit bestimmten Zusatzstoffen oder ein anderes Fluid als Wasser, insbesondere Öl, sein.

Die Erfindung wird vorliegend am Beispiel eines Temperiergerätes zur Verwendung mit einer wasserdurchströmten Temperier-Manschette zum Auflegen auf die betroffenen Körperbereiche des Patienten beschrieben. Dies stellt jedoch keine Einschränkung dar; das erfindungsgemäße medizinische Temperiergerät lässt sich auch für andere Anwendungen einsetzen, beispielsweise zur Verwendung mit einem Temperierkörper, welcher in eine Körperöffnung des Patienten eingeführt wird.

Die vorliegende Erfindung bedeutet eine Abkehr von bisher verwendeten Konstruktionen für derartige Temperiergeräte. Diese arbeiten oftmals mit Kompressor-Temperiereinrichtungen und Kältemitteln, deren Handhabung bestimmten Einschränkungen unterliegt.

Die von der Erfindung zur Verwendung in den Temperiereinheiten vorgeschlagenen thermoelektrischen Elemente haben dagegen keine mechanischen oder hydraulischen Bauteile und sind daher einfach in der Handhabung, zuverlässig im Betrieb, praktisch wartungsfrei und kostengünstig herzustellen. Auch muss der Benutzer beim Transport, bei der Inbetriebnahme und Verwendung des Temperiergerätes nicht dessen räumliche Orientierung beachten. Diese Vorteile tragen insgesamt dazu bei, den Komfort des Benutzers des Temperiergerätes zu erhöhen.

Derartige Temperiereinheiten, welche ein thermoelektrisches Element, ein Wärmetauscherelement und einen Kühlkörper aufweisen, werden bereits als fertige Module, sogenannte "Liquid-to-Air-Module", am Markt angeboten. Hierbei werden als thermoelektrische Elemente vorzugsweise die aus bestimmten Halbleitern bestehenden, sogenannten "Peltier-Elemente" eingesetzt.

Die Temperierleistung eines thermoelektrischen Elements beruht darauf, dass beim Anlegen einer Spannung an die elektrischen Anschlüsse des Elements zwischen gegenüberliegenden Oberflächen des Elements eine Temperaturdifferenz erzeugt wird, d. h. es wird Wärme von der einen zu der anderen Oberfläche transportiert. Es ist daher zweckmäßig, auf einer dieser Oberflächen das Wärmetauscherelement und auf der anderen Oberfläche den Kühlkörper anzuordnen.

Im Folgenden wird der Einsatz des medizinischen Temperiergerätes für eine Kälteanwendung betrachtet, d. h. die vorbestimmte Temperatur, mit der das Fluid aus dem Gehäuse herausgeführt wird, soll geringer sein als die Ausgangstemperatur, mit der das Fluid von der Fluidversorgungseinrichtung bereitgestellt wird.

In diesem Fall wird das thermoelektrische Element so geschaltet, dass Wärme von der zum Wärmetauscherelement hin orientierten Oberfläche zu der zum Kühlkörper hin orientierten Oberfläche transportiert wird. Auf diese Weise kann dem Fluid über das Wärmetauscherelement an der entsprechenden Oberfläche des thermoelektrischen Elements Wärme entzogen und diese Wärme auf der anderen Oberfläche des thermoelektrischen Elements an den Kühlkörper abgegeben werden, so dass das Fluid abgekühlt und der Kühlkörper erwärmt wird.

Für die Leistung der gesamten Temperiereinheit ist entscheidend, in welchem Maße die im Kühlkörper entstehende Wärme wieder in die Umgebung abgeführt wird, da hiervon die am thermoelektrischen Element entstehende Temperaturdifferenz abhängt.

Hierzu schlägt die Erfindung nun vor, die beiden Temperiereinheiten derart anzuordnen, dass ihre Kühlkörper einander zugewandt sind. Dadurch ergibt sich eine kompakte Anordnung der Temperiereinheiten und somit ein relativ kleines Temperiergerät, was insbesondere für einen mobilen Einsatz, insbesondere für Heimanwendungen, von Vorteil ist. Außerdem lassen sich - insbesondere, wenn die Kühlkörper Rippen aufweisen - durch die gegenseitige Anordnung der Kühlkörper zwischen diesen Rippen geschlossener Kühlkanäle bilden, aus denen sich die Wärme, insbesondere durch einen genau definierten und gezielt in die Kühlkanäle eingebrachten Luftstrom, besonders gut abführen lässt.

In einer bevorzugten Ausführung der Erfindung weist die Fluidversorgungseinrichtung eine Fluidzuströmungseinrichtung auf, durch welche das Fluid von außen in das Gehäuse eingeführt wird.

Dabei ist die Fluidzuströmungseinrichtung vorzugsweise zur Einführung wenigstens eines Teils des durch die Fluidabströmungseinrichtung aus dem Gehäuse herausgeführten Fluids in das Gehäuse vorgesehen, so dass sich ein wenigstens teilweiser Fluidkreislauf ergibt.

Dabei weist das durch die Fluidzuströmungseinrichtung in das Gehäuse eingeführte Fluid vorzugsweise eine andere Temperatur, und zwar vorzugsweise eine geringere Temperatur (im Falle des Einsatzes des Temperiergerätes zur Wärmebehandlung) und weiter vorzugsweise eine höhere Temperatur (im Falle des Einsatzes des Temperiergerätes zur Kältebehandlung), und/oder einen anderen, vorzugsweise geringeren, Druck auf als das durch die Fluidabströmungseinrichtung aus dem Gehäuse herausgeführte Fluid.

In einer weiteren bevorzugten Ausführung der Erfindung weist die Fluidversorgungseinrichtung einen im Gehäuse angeordneten Fluidbehälter auf. Somit kann der Benutzer das Temperiergerät auf bequeme Weise bei der Inbetriebnahme mit Fluid befüllen.

In einer weiteren bevorzugten Ausführung der Erfindung ist die Fluidzuströmungseinrichtung mit dem Fluidbehälter fluidleitend verbunden. Auf diese Weise kann der Fluidbehälter in den Fluidkreislauf eingebunden werden und dabei als Puffer für die zirkulierende Fluidmenge dienen, wobei eine beispielsweise durch Leckagen oder Verdunstung verloren gehende Fluidmenge automatisch ausgeglichen wird.

In einer weiteren bevorzugten Ausführung der Erfindung sind die erste Temperiereinheit und die zweite Temperiereinheit im Wesentlichen gleich aufgebaut. Dies vereinfacht die Konstruktion des Temperiergerätes sowie die Logistik und damit die Kosten bei dessen Herstellung.

In einer weiteren bevorzugten Ausführung der Erfindung weisen der Kühlkörper der ersten Temperiereinheit und der Kühlkörper der zweiten Temperiereinheit jeweils einen Bereich mit Kühlrippen auf, und diese Bereiche mit Kühlrippen sind einander zugewandt.

Die Kühlrippen des Kühlkörpers der ersten Temperiereinheit und/oder die Kühlrippen des Kühlkörpers der zweiten Temperiereinheit sind dabei vorzugsweise im sogenannten "Skived-Fin"-Verfahren hergestellt.

Bei diesem Verfahren wird ein massiver, annähernd quaderförmiger Metallblock mit einer Vielzahl von schräg zur Oberfläche gerichteten Schnitten in parallelen Ebenen bis zu einer bestimmten Tiefe, insbesondere bis kurz vor die gegenüberliegende Seite des Blockes, versehen. Sodann werden die hierbei zwischen benachbarten Schnitten entstandenen Metallscheiben aufgerichtet, so dass sie eng benachbarte, voneinander nur geringfügig beabstandete Rippen bilden, welche senkrecht zur gegenüberliegenden Seitenfläche des Blockes stehen. Diese gegenüberliegende Seitenfläche bildet eine durchgehende, ebene Oberfläche des Kühlkörpers, an welcher das zu kühlende Element befestigt wird.

Auf diese Weise lässt sich in einem nicht-spanenden und nicht-abtragenden Herstellungsverfahren ein Kühlkörper aus einem Stück und damit ohne Materialübergänge mit einer Vielzahl von eng nebeneinander stehenden Kühlrippen fertigen, wodurch sich eine entsprechend hohe Kühlleistung des Kühlkörpers erreichen lässt.

In einer weiteren bevorzugten Ausführung der Erfindung sind durch den Kühlkörper der ersten Temperiereinheit gemeinsam mit dem Kühlkörper der zweiten Temperiereinheit, vorzugsweise geschlossene, Kühlkanäle gebildet. Dabei kann man sich die oben beschriebene Ausführung der Kühlkörper mit, insbesondere einseitig offenen, Kühlrippen zu Nutze machen. Zwischen den beiden einander zugewandten Kühlkörpern bilden sich bei geeigneter Orientierung der Kühlrippen dann Kühlkanäle. Aus derartigen, insbesondere geschlossenen, Kühlkanälen kann die Wärme aus den Kühlkörpern, insbesondere durch einen auf die beiden Temperiereinheiten gemeinsam gerichteten Luftstrom, besonders gut abgeführt werden.

In einer weiteren bevorzugten Ausführung der Erfindung sind die erste Temperiereinheit und die zweite Temperiereinheit im Wesentlichen spiegelsymmetrisch zueinander angeordnet. Auf diese Weise lassen sich die genannten Kühlkanäle durch die beiden Kühlkörper besonders einfach bilden, insbesondere, wenn jeweils eine Scheitelfläche einer Kühlrippe des einen Kühlkörpers im Wesentlichen mit einer Scheitelfläche einer Kühlrippe des anderen Kühlkörpers in Deckung kommt, wodurch sich ohne weitere konstruktive Maßnahmen eine durchgehende Wand eines derartigen Kühlkanals ergibt.

In einer weiteren bevorzugten Ausführung der Erfindung weist die Temperiereinrichtung wenigstens eine Ventilationseinrichtung auf, die einen Luftstrom im Gehäuse bewirkt, der die erste Temperiereinheit und die zweite Temperiereinheit berührt.

Die Ventilationseinrichtung weist vorzugsweise einen, besonders bevorzugt jedoch mehrere, und zwar insbesondere zwei, Lüfter auf, welche auf einander gegenüberliegenden Seiten der Einheit der Kühlkörper der ersten und der zweiten Temperiereinheit angeordnet sind. Die Lüfter sind vorzugsweise so geschaltet, dass wenigstens ein Lüfter Kühlluft in zwischen den Kühlkörpern gebildete Kühlkanäle hineinbläst und wenigstens ein anderer Lüfter die Luft aus den Kühlkanälen wieder absaugt. Damit lässt sich eine starker Luftstrom durch die Kühlkanäle realisieren und somit eine gute Wärmeabfuhr von den Kühlkörpern erreichen.

In einer weiteren bevorzugten Ausführung der Erfindung verläuft der Luftstrom im Wesentlichen senkrecht zu einer Richtung, in der der Kühlkörper der ersten Temperiereinheit und der Kühlkörper der zweiten Temperiereinheit einander zugewandt sind. Dieser Verlauf des Luftstroms resultiert in einer besonders geringen Länge des Luftstroms durch die beiden Kühlkörper und damit zu einer guten Wärmeabführung.

Falls die erste Temperiereinheit und die zweite Temperiereinheit im Wesentlichen spiegelsymmetrisch zueinander angeordnet sind, verläuft der Luftstrom besonders bevorzugt im Wesentlichen parallel zu einer Symmetrieebene der ersten Temperiereinheit und der zweiten Temperiereinheit.

In einer weiteren bevorzugten Ausführung der Erfindung sind das Wärmetauscherelement der ersten Temperiereinheit und das Wärmetauscherelement der zweiten Temperiereinheit bezüglich der Fluiddurchströmung in Reihe geschaltet. Durch diese Kombination lassen sich die Wärmetauscherkapazitäten der beiden Temperiereinheiten besonders gut ausnutzen, da die in den beiden Temperiereinheiten erzeugten Temperaturdifferenzen additiv zur Kühlung des Fluids eingesetzt werden können.

Die Erfindung wird im Folgenden anhand eines Ausführungsbeispiels unter Bezugnahme auf die beigefügten Zeichnungen erläutert. Dabei zeigen:

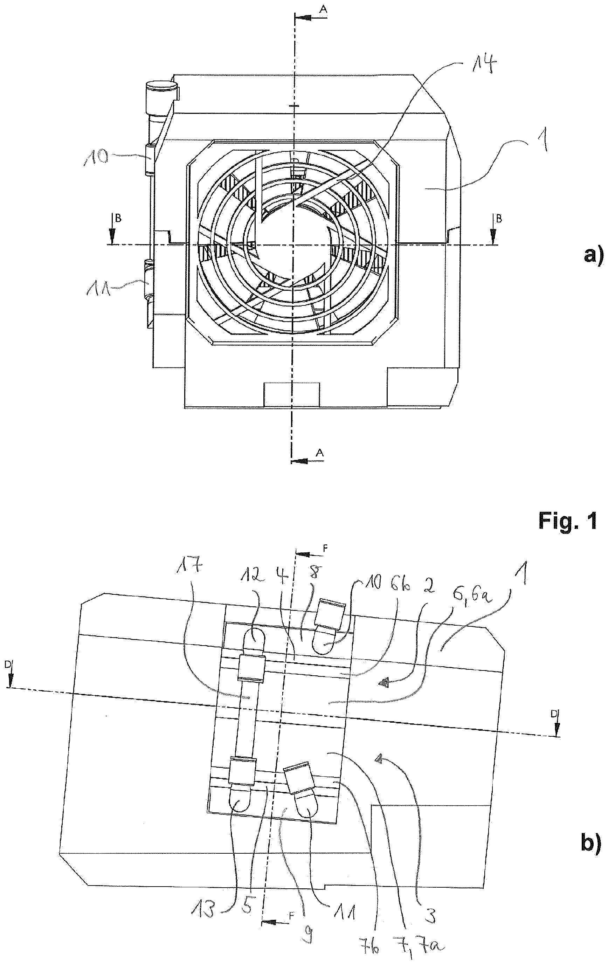

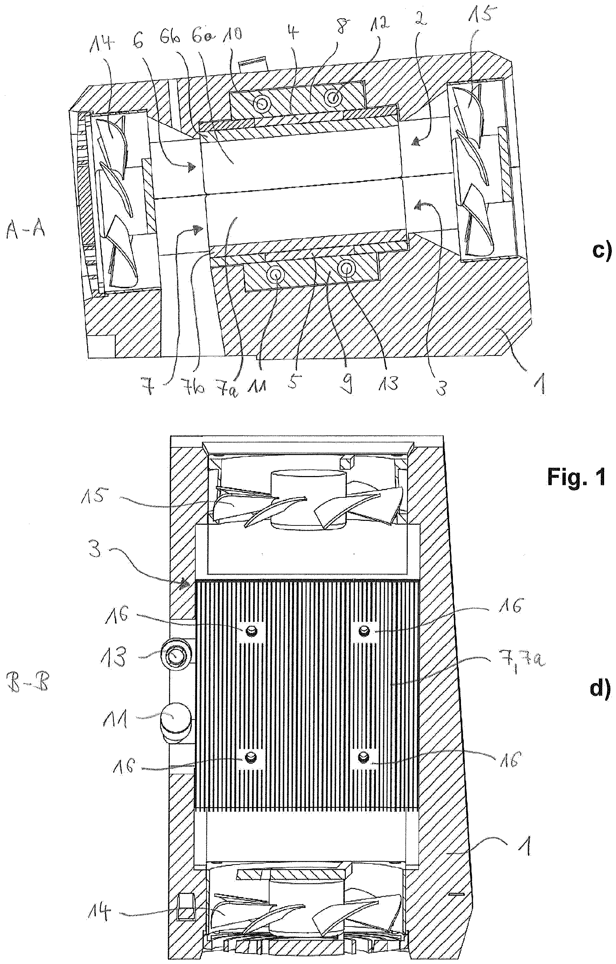

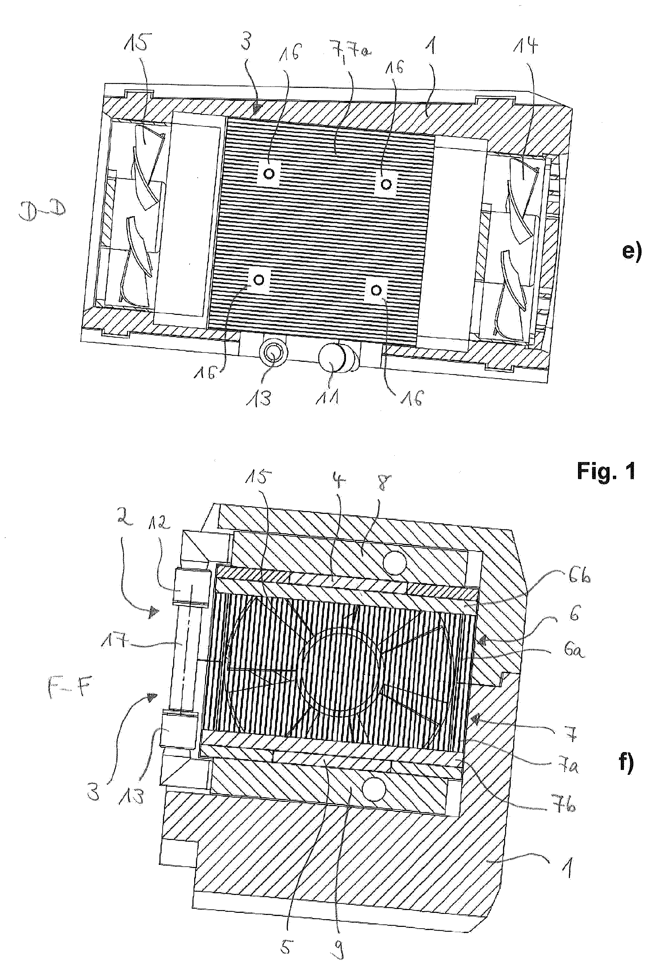

- Fig. 1:: mehrere Ansichten einer Temperiereinrichtung für ein erfindungsgemäßes Temperiergerät, nämlich:
a) eine Ansicht von vorne,
b) eine Ansicht von der Seite,
c) eine Schnittansicht von der Seite entlang der Linie A-A in Fig. 1 a),
d) eine Schnittansicht von oben entlang der Linie B-B in Fig. 1 a),
e) eine Schnittansicht von oben entlang der Linie D-D in Fig. 1 b),
f) eine Schnittansicht von vorne entlang der Linie F-F in Fig. 1 b);

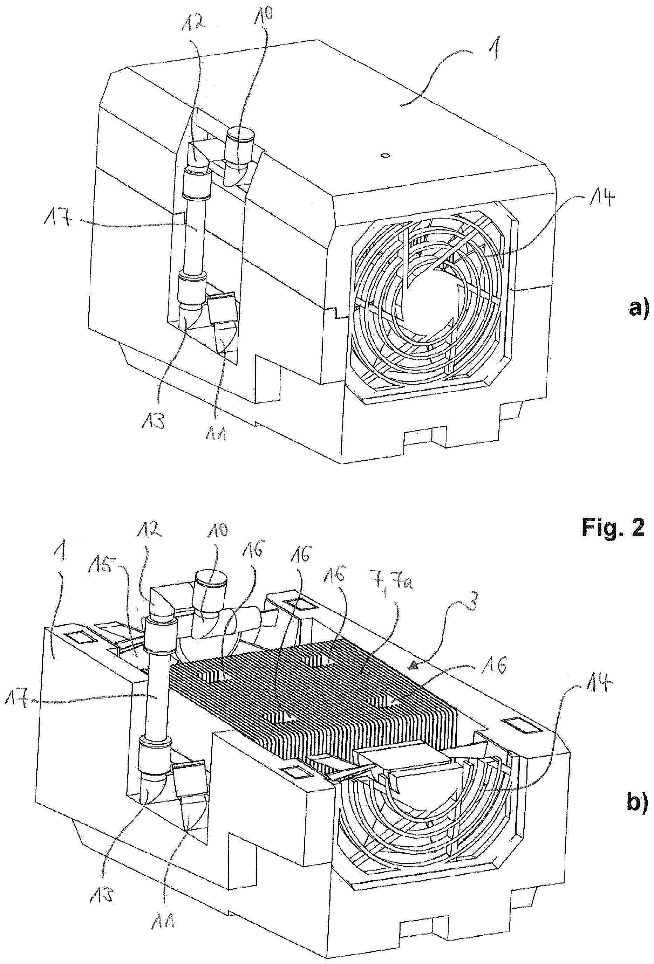

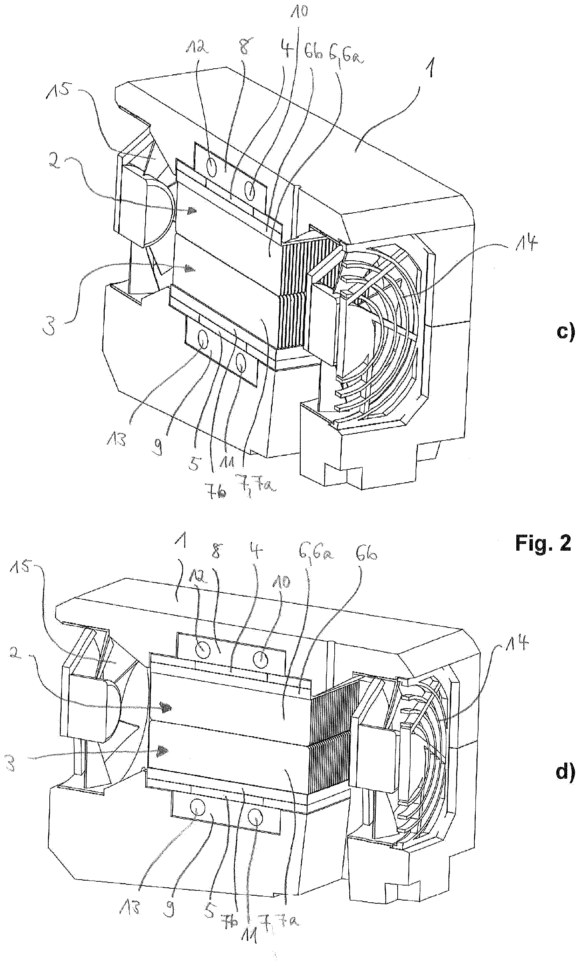

- Fig. 2:: mehrere perspektivische Ansichten einer Temperiereinrichtung für ein erfindungsgemäßes Temperiergerät, nämlich:
a) eine Ansicht von schräg vorne,
b) eine Ansicht von schräg vorne einer horizontal aufgeschnittenen Temperiereinrichtung,
c) eine Ansicht von schräg vorne einer vertikal aufgeschnittenen Temperiereinrichtung,
d) eine Ansicht von der Seite einer vertikal aufgeschnittenen Temperiereinrichtung.

Die einzelnen Ansichten in den Fig. 1 und 2 stellen jeweils dieselbe Ausführungsform der Temperiereinrichtung dar. Entsprechend bezeichnen gleiche Bezugszeichen in den Figuren auch gleiche Elemente dieser Ausführungsform. In der folgenden Beschreibung werden daher die Figuren nicht einzeln erläutert, sondern nur die in den Figuren dargestellte Ausführungsform als Ganzes.

Die in den Figuren dargestellte Temperiereinrichtung ist zum Einbau in ein erfindungsgemäßes Temperiergerät vorgesehen, welches neben der Temperiereinrichtung selbst eine Fluidversorgungseinrichtung, eine Fluidabströmungseinrichtung und eine Pumpeneinrichtung aufweist. Letztere sind jedoch in den Figuren nicht dargestellt.

Der Fluidkreislauf während des Betriebs des erfindungsgemäßen Temperiergerätes verläuft vorzugsweise wie folgt:
Das von der Pumpeneinrichtung kommende und dort mit Druck beaufschlagte Fluid strömt zunächst zu der Temperiereinrichtung, welche im Folgenden noch näher beschrieben wird, und durch diese hindurch, wo es temperiert, d. h. erwärmt oder abgekühlt, wird.

Von dort fließt das Fluid zur Fluidabströmungseinrichtung und aus dem Temperiergerät heraus. An die Fluidabströmungseinrichtung ist vorzugsweise eine Manschette zur Auflage auf einen Körperteil eines Patienten, insbesondere auf eine Hand, auf ein Knie oder auf einen anderen Körperteil, angeschlossen, welcher temperiert werden soll. An diesen Körperteil wird Wärmeenergie abgegeben bzw. von diesem wird Wärmeenergie entzogen, je nachdem, ob eine Wärme- oder Kältebehandlung durchgeführt werden soll.

Eine an das Temperiergerät anzuschließende Manschette hat typischerweise einen Fluidinhalt von bis zu 0,5 Liter. Vorzugsweise wird lediglich eine Manschette an das Temperiergerät angeschlossen. Bei entsprechender Auslegung des Temperiergerätes können jedoch auch mehrere Manschetten angeschlossen werden.

Von der Manschette fließt das Fluid zurück in das Temperiergerät zu der Fluidversorgungseinrichtung, insbesondere in einen Fluidbehälter, und von dort zur Pumpeneinrichtung. Dort wird das Fluid wieder mit Druck beaufschlagt, und der beschriebene Fluidkreislauf beginnt erneut. Selbstverständlich lassen sich die einzelnen, an dem Kreislauf beteiligten Einrichtungen auch in einer anderen Reihenfolge anordnen.

Die Temperiereinrichtung weist eine erste Temperiereinheit 2 und eine zweite Temperiereinheit 3 auf, welche von einem gemeinsamen Gehäuse 1 umschlossen sind. Das Gehäuse 1 kann dabei zweiteilig ausgeführt sein, damit die Temperiereinheiten 2, 3 in das Gehäuse 1 eingesetzt werden können.

Die beiden Temperiereinheiten 2, 3 sind als sogenannte "Liquid-to-Air-Module" ausgeführt. Im Ausführungsbeispiel sind die Temperiereinheiten 2, 3 baugleich. Sie können jedoch auch unterschiedlich gestaltet sein.

Die Temperiereinheiten 2, 3 weisen jeweils ein Peltier-Element 4, 5 auf, welches jeweils zwischen einem Kühlkörper 6, 7 und einem Wärmetauscher 8, 9 angeordnet ist und diese auf seinen gegenüberliegenden Seiten jeweils flächig berührt.

Die Kühlkörper 6, 7 weisen jeweils eine Vielzahl (im Ausführungsbeispiel etwa 50) von rechteckigen, parallel zueinander angeordneten und voneinander beabstandeten Rippen 6a, 7a auf. Die Rippen stehen jeweils senkrecht auf einer Bodenplatte 6b, 7b. Die Kühlkörper sind vorzugsweise im sogenannten "Skived-Fin"-Verfahren hergestellt.

Wie in den Fig. 1 d), 1 e) und 2 b) zu erkennen, weisen die Kühlkörper 6, 7 im Bereich ihrer Rippen 6a, 7a jeweils vier quadratische und gemeinsam ebenfalls die Ecken eines Quadrats bildende Aussparungen 16 auf. Die Anzahl und die geometrische Anordnung der Aussparungen 16 können natürlich auch anders gewählt werden. In den Aussparungen 16 sind Befestigungselemente, vorzugsweise Schrauben, Stifte oder Nieten, zur Befestigung des Kühlkörpers 6, 7 an dem jeweiligen Peltier-Element 4, 5 und vorzugsweise auch an dem jeweiligen Wärmetauscher 8, 9 angeordnet.

Im Falle von Schrauben als Befestigungselementen führen diese vorzugsweise von den Aussparungen 16 aus ggf. durch Durchgangsbohrungen in den Peltier-Elementen 4, 5 zu den Wärmetauschern 8, 9 und sind in entsprechenden Gewinden in den Peltier-Elementen 4, 5 und/oder in den Wärmetauschern 8, 9 eingeschraubt.

Die Aussparungen 16 können bereits vor dem Herstellen der Rippen 6a, 7a in die Kühlkörper 6, 7, vorzugsweise spanend, weiter vorzugsweise durch Bohren oder Ausfräsen, eingebracht werden.

Weiter vorzugsweise können die Befestigungselemente, vorzugsweise Schrauben, jedoch auch von den Wärmetauschern 8, 9 aus ggf. durch Durchgangsbohrungen in den Peltier-Elementen 4, 5 zu den Kühlkörpern 6, 7 führen und in entsprechenden Gewinden in den Peltier-Elementen 4, 5 und/oder in den Kühlkörpern 6, 7 eingeschraubt sein.

In diesem Fall sind die Aussparungen 16 in den Kühlkörpern 6, 7 nicht mehr nötig, wodurch eine Kosteneinsparung erzielt wird. Außerdem sind die Kühlrippen 6a, 7a nicht unterbrochen, und es ragen keine Schraubenköpfe in den Luftstrom hinein, wodurch mehr Kühloberfläche und damit mehr Kühlleistung zur Verfügung steht. Schließlich kann bei einer Verschraubung von den Wärmetauschern 8, 9 aus mit Hilfe von thermischen Trennelementen, insbesondere Isolierscheiben und -hülsen, eine bessere thermische Trennung zwischen der jeweiligen Kalt- und Warmseite der Temperiereinheiten 2, 3 erreicht werden.

Die Wärmetauscher 8, 9 weisen jeweils einen vorzugsweise weitgehend oder vollständig massiven Metallblock auf, durch welchen jeweils eine (in den Figuren nicht sichtbare) interne Fluidleitung für das zu temperierende Fluid führt. Die interne Fluidleitung kann insbesondere in Schlangenlinien oder spiralförmig sowie in einer oder in mehreren Ebenen durch den Metallblock verlaufen. Die interne Fluidleitung in der ersten Temperiereinheit 2 endet an den Anschlüssen 10 und 12 und die interne Fluidleitung in der zweiten Temperiereinheit 3 an den Anschlüssen 11 und 13.

Die beiden Wärmetauscher 8, 9 und damit die Temperiereinheiten 2, 3 sind in Reihe geschaltet, wie in den Fig. 1 b) und 2 a) am besten zu erkennen ist:
Das Fluid strömt durch den Anschluss 11 in die zweite Temperiereinheit 3 ein, durchläuft die interne Fluidleitung des Wärmetauschers 9 der zweiten Temperiereinheit 3 und tritt an der Öffnung 13 des Wärmetauschers 9 wieder aus. An dem Anschluss 13 ist eine Fluidverbindungsleitung 17 angeschlossen, welche die zweite Temperiereinheit 3 mit der ersten Temperiereinheit 2 verbindet. Durch die Fluidverbindungsleitung 17 fließt das Fluid zum Anschluss 12 an der ersten Temperiereinheit 2, durchläuft die interne Fluidleitung des Wärmetauschers 8 der ersten Temperiereinheit 2 und tritt am Anschluss 10 des Wärmetauschers 8 wieder aus.

In der Vorderseite und in der Rückseite des Gehäuses 1 ist jeweils ein Lüfter 14, 15 angeordnet, welcher jeweils als herkömmlicher Lüfter mit einem Turbinenrad ausgeführt ist. Es sind jedoch auch andere Bauformen für die Lüfter möglich. Die Rotationsachsen der Turbinenräder der beiden Lüfter 14,15 liegen auf einer Geraden. Die Lüfter 14, 15 erzeugen im Betrieb des Temperiergerätes einen im Wesentlichen in axialer Richtung, d. h. parallel zur Linie D-D in Fig. 1 b), durch das Gehäuse 1 verlaufenden Luftstrom.

Die Temperiereinheiten 2, 3 sind etwa in der Mitte des Gehäuses 1 derart angeordnet, dass der Kühlkörper 6 der ersten Temperiereinheit 2 nach unten und der Kühlkörper 7 der zweiten Temperiereinheit 3 nach oben weist, so dass die Kühlkörper 6, 7 sich an den offenen Enden ihrer Rippen 6a, 7a berühren. Vorzugsweise fällt dabei jeweils eine Scheitellinie einer Rippe des Kühlkörpers 6 mit einer Scheitellinie einer Rippe des Kühlkörpers 7 geometrisch zusammen. Dadurch ergeben sich über die gesamte axiale Erstreckung der Kühlkörper 6, 7 verlaufende, geschlossene Kühlkanäle, welche von dem genannten Luftstrom, vorzugsweise parallel zu der Trennebene zwischen den Kühlkörpern 6, 7, durchströmt werden.

Hierdurch ergibt sich eine besonders gute Abführung der Wärme, welche jeweils an den Peltier-Elementen 4, 5 entsteht und in die Kühlkörper 6, 7 geleitet wird. Dadurch lässt sich an den Peltier-Elementen 4, 5 auch eine große Temperaturdifferenz erzeugen. Die erreichbare Temperaturdifferenz hängt weiterhin auch von der Größe der an das Temperiergerät angeschlossenen Manschette ab, da diese aufgrund der Abgabe oder Aufnahme von Wärme an den bzw. von dem Körper des Patienten als weiterer Wärmetauscher fungiert. Je größer die Manschette ist, desto stärker ist auch deren Wärmetauscherleistung. Die an jedem der Peltier-Elemente 4, 5 erreichbare Temperaturdifferenz beträgt vorzugsweise 11 Kelvin oder mehr.

Für eine medizinische Wärme- oder Kältebehandlung ist es wünschenswert, die Temperatur, mit der das Fluid das Temperiergerät verlässt, möglichst konstant zu halten, bei einer Kältebehandlung beispielsweise auf 15 °C. Die hierfür notwendige Temperaturregelung kann auf einfache Weise dadurch vorgenommen werden, dass ein (nicht dargestellter) Temperatursensor die Temperatur des abströmenden Fluids misst und dass ein oder beide Peltier-Elemente 4, 5 ein- bzw. ausgeschaltet werden, sobald die gemessene Temperatur je nach Anwendungsfall unter oder über der Solltemperatur liegt.

Insbesondere durch die Möglichkeit, nur eines der beiden Peltier-Elemente 4, 5 ein- und auszuschalten, kann hierbei eine noch genauere Regelung erreicht werden, als wenn beide Peltier-Elemente 4, 5 immer gleichzeitig ein- bzw. ausgeschaltet werden.

Auch kann durch ein periodisches Ein- und Ausschalten eines oder beider Peltier-Elemente 4, 5 mit einem bestimmten Tastverhältnis, d. h. mit einem bestimmten Verhältnis zwischen der Länge der Ein- und der Ausschaltperiode, die Stärke der Temperierleistung der Temperiereinrichtung gesteuert oder geregelt werden.

### Bezugszeichenliste

- 1: Gehäuse
- 2: Erste Temperiereinheit
- 3: Zweite Temperiereinheit
- 4,5: Peltier-Element
- 6, 7: Kühlkörper
- 6a, 7a: Rippen des Kühlkörpers
- 6b, 7b: Bodenplatte des Kühlkörpers
- 8, 9: Wärmetauscher
- 10-13: Anschlüsse der Fluidleitungen
- 14, 15: Lüfter
- 16: Aussparung im Kühlkörper
- 17: Fluidverbindungsleitung

## Patentansprüche

1. Medizinisches Temperiergerät mit
wenigstens einem Gehäuse (1),
wenigstens einer Fluidversorgungseinrichtung, durch welche ein Fluid in dem Gehäuse (1) bereitgestellt wird,
wenigstens einer im Gehäuse (1) angeordneten Temperiereinrichtung, die das durch das Gehäuse (1) strömende Fluid temperiert,
wenigstens einer Fluidabströmungseinrichtung, durch welche das Fluid mit einer im Wesentlichen vorbestimmten Temperatur aus dem Gehäuse (1) herausgeführt wird, und
wenigstens einer im Gehäuse (1) angeordneten Pumpeneinrichtung, welche das durch das Gehäuse (1) strömende Fluid mit Druck beaufschlagt,
wobei diese wenigstens eine Temperiereinrichtung (2, 3) wenigstens eine erste Temperiereinheit (2) und wenigstens eine zweite Temperiereinheit (3) aufweist,
welche jeweils wenigstens ein thermoelektrisches Element (4, 5), insbesondere wenigstens ein Peltier-Element, wenigstens ein von Fluid durchströmbares Wärmetauscherelement (8, 9) zur Übertragung von Wärme auf das oder von dem Fluid und wenigstens einen Kühlkörper (6, 7) aufweisen,
**dadurch gekennzeichnet,**
**dass** der wenigstens eine Kühlkörper (6) der wenigstens einen ersten Temperiereinheit (2) und der wenigstens eine Kühlkörper (7) der wenigstens einen zweiten Temperiereinheit (3) einander zugewandt sind und dass durch den Kühlkörper (6) der ersten Temperiereinheit (2) gemeinsam mit dem Kühlkörper (7) der zweiten Temperiereinheit (3), vorzugsweise geschlossene, Kühlkanäle gebildet sind.

2. Medizinisches Temperiergerät gemäß Anspruch 1, wobei die Fluidversorgungseinrichtung eine Fluidzuströmungseinrichtung aufweist, durch welche das Fluid von außen in das Gehäuse (1) eingeführt wird.

3. Medizinisches Temperiergerät gemäß Anspruch 2, wobei die Fluidzuströmungseinrichtung zur Einführung wenigstens eines Teils des durch die Fluidabströmungseinrichtung aus dem Gehäuse (1) herausgeführten Fluids in das Gehäuse (1) vorgesehen ist, so dass sich ein wenigstens teilweiser Fluidkreislauf ergibt, wobei das durch die Fluidzuströmungseinrichtung in das Gehäuse (1) eingeführte Fluid eine andere, vorzugsweise geringere, weiter vorzugsweise höhere, Temperatur und/oder einen anderen, vorzugsweise geringeren, Druck aufweist als das durch die Fluidabströmungseinrichtung aus dem Gehäuse (1) herausgeführte Fluid.

4. Medizinisches Temperiergerät gemäß einem der vorhergehenden Ansprüche, wobei die Fluidversorgungseinrichtung einen im Gehäuse (1) angeordneten Fluidbehälter aufweist.

5. Medizinisches Temperiergerät gemäß den Ansprüchen 2 und 4, wobei die Fluidzuströmungseinrichtung mit dem Fluidbehälter fluidleitend verbunden ist.

6. Medizinisches Temperiergerät gemäß einem der vorhergehenden Ansprüche, wobei die erste Temperiereinheit (2) und die zweite Temperiereinheit (3) im Wesentlichen gleich aufgebaut sind.

7. Medizinisches Temperiergerät gemäß einem der vorhergehenden Ansprüche, wobei der Kühlkörper (6) der ersten Temperiereinheit (2) und der Kühlkörper (7) der zweiten Temperiereinheit (3) jeweils einen Bereich mit Kühlrippen (6a, 7a) aufweist und dass diese Bereiche mit Kühlrippen (6a, 7a) einander zugewandt sind.

8. Medizinisches Temperiergerät gemäß Anspruch 7, wobei die Kühlrippen (6a) des Kühlkörpers (6) der ersten Temperiereinheit (2) und/oder die Kühlrippen (7a) des Kühlkörpers (7) der zweiten Temperiereinheit (3) im "Skived-Fin"-Verfahren hergestellt sind.

9. Medizinisches Temperiergerät gemäß einem der vorhergehenden Ansprüche, wobei die erste Temperiereinheit (2) und die zweite Temperiereinheit (3) im Wesentlichen spiegelsymmetrisch zueinander angeordnet sind.

10. Medizinisches Temperiergerät gemäß einem der vorhergehenden Ansprüche, wobei die Temperiereinrichtung (2, 3) wenigstens eine Ventilationseinrichtung (14, 15) aufweist, die einen Luftstrom im Gehäuse (1) bewirkt, der die erste Temperiereinheit (2) und die zweite Temperiereinheit (3) berührt.

11. Medizinisches Temperiergerät gemäß Anspruch 10, wobei der Luftstrom im Wesentlichen senkrecht zu einer Richtung verläuft, in der der Kühlkörper (6) der ersten Temperiereinheit (2) und der Kühlkörper (7) der zweiten Temperiereinheit (3) einander zugewandt sind.

12. Medizinisches Temperiergerät gemäß Anspruch 9 und gemäß Anspruch 10 oder 11, wobei der Luftstrom im Wesentlichen parallel zu einer Symmetrieebene der ersten Temperiereinheit (2) und der zweiten Temperiereinheit (3) verläuft.

13. Medizinisches Temperiergerät gemäß einem der vorhergehenden Ansprüche, wobei das Wärmetauscherelement (8) der ersten Temperiereinheit (2) und das Wärmetauscherelement (9) der zweiten Temperiereinheit (3) bezüglich der Fluiddurchströmung in Reihe geschaltet sind.

## Claims

1. Medical temperature control apparatus with
at least one housing (1),
at least one fluid supply device by which a fluid is provided in the housing (1), at least one temperature control device arranged in the housing (1),
which controls the temperature of the fluid flowing through the housing (1),
at least one fluid outflow device by which the fluid is led out of the housing (1) at a substantially predetermined temperature, and
at least one pump device arranged in the housing (1) which pressurizes the fluid flowing through the housing (1),
wherein the at least one temperature control device (2, 3) comprises at least one first temperature control unit (2) and at least one second temperature control unit (3),
each comprising at least one thermoelectric element (4, 5), in particular at least one Peltier element, at least one heat exchanger element (8, 9) through which fluid can flow for transferring heat to or from the fluid, and at least one heat sink (6, 7),
**characterized in that**
the at least one heat sink (6) of the at least one first temperature control unit (2) and the at least one heat sink (7) of the at least one second temperature control unit (3) face one another, and that, preferably closed, cooling channels are formed by the heat sink (6) of the first temperature control unit (2) together with the heat sink (7) of the second temperature control unit (3).

2. Medical temperature control apparatus according to claim 1, wherein
the fluid supply device comprises a fluid inflow device by which the fluid is introduced into the housing (1) from the outside.

3. Medical temperature control apparatus according to claim 2, wherein
the fluid inflow device is provided for introducing at least a part of the fluid led out of the housing (1) by the fluid outflow device into the housing (1), such that an at least partial fluid circuit results, wherein the fluid introduced into the housing (1) by the fluid inflow device has a different, preferably lower, more preferably higher, temperature and/or a different, preferably lower, pressure than the fluid led out of the housing (1) by the fluid outflow device.

4. Medical temperature control apparatus according to any one of the preceding claims, wherein the fluid supply device comprises a fluid container arranged in the housing (1).

5. Medical temperature control apparatus according to claims 2 and 4, wherein the fluid inflow device is connected to the fluid container in a fluid-conducting manner.

6. Medical temperature control apparatus according to any one of the preceding claims, wherein the first temperature control unit (2) and the second temperature control unit (3) are constructed substantially identically.

7. Medical temperature control apparatus according to any one of the preceding claims, wherein the heat sink (6) of the first temperature control unit (2) and the heat sink (7) of the second temperature control unit (3) each comprises an area with cooling fins (6a, 7a) and that these areas with cooling fins (6a, 7a) face each other.

8. Medical temperature control apparatus according to claim 7, wherein the cooling fins (6a) of the heat sink (6) of the first temperature control unit (2) and/or the cooling fins (7a) of the heat sink (7) of the second temperature control unit (3) are manufactured in the "skived-fin" process.

9. Medical temperature control apparatus according to any one of the preceding claims, wherein the first temperature control unit (2) and the second temperature control unit (3) are arranged substantially axially symmetrically to one another.

10. Medical temperature control apparatus according to any one of the preceding claims, wherein the temperature control device (2, 3) comprises at least one ventilation device (14, 15) which causes an air flow in the housing (1) which contacts the first temperature control unit (2) and the second temperature control unit (3).

11. Medical temperature control apparatus according to claim 10, wherein the air flow is substantially perpendicular to a direction in which the heat sink (6) of the first temperature control unit (2) and the heat sink (7) of the second temperature control unit (3) face each other.

12. Medical temperature control apparatus according to claim 9 and according to claim 10 or 11, wherein the air flow is substantially parallel to a symmetry plane of the first temperature control unit (2) and the second temperature control unit (3).

13. Medical temperature control apparatus according to any one of the preceding claims, wherein the heat exchanger element (8) of the first temperature control unit (2) and the heat exchanger element (9) of the second temperature control unit (3) are connected in series with respect to the fluid flow.

## Revendications

1. Appareil médical de thermorégulation avec
au moins un boîtier (1),
au moins un dispositif d'alimentation en fluide, par lequel un fluide est fourni dans le boîtier (1),
au moins un dispositif de thermorégulation disposé dans le boîtier (1), qui thermorégule le fluide circulant à travers le boîtier (1),
au moins un dispositif d'évacuation de fluide, par lequel le fluide est guidé à l'extérieur du boîtier (1) avec une température sensiblement prédéfinie, et
au moins un dispositif de pompage disposé dans le boîtier (1), lequel soumet à l'action d'une pression le fluide circulant à travers le boîtier (1),
dans lequel ledit au moins un dispositif de thermorégulation (2, 3) présente au moins une première unité de thermorégulation (2) et au moins une deuxième unité de thermorégulation (3), lesquelles présentent respectivement au moins un élément thermoélectrique (4, 5), en particulier au moins un élément Peltier, au moins un élément échangeur de chaleur (8, 9) pouvant être traversé par du fluide pour transférer de la chaleur sur le fluide ou depuis celui-ci, et au moins un corps de refroidissement (6, 7),
**caractérisé en ce**
**que** l'au moins un corps de refroidissement (6) de l'au moins une première unité de thermorégulation (2) et l'au moins un corps de refroidissement (7) de l'au moins une deuxième unité de thermorégulation (3) sont tournés l'un vers l'autre, et que des canaux de refroidissement, de préférence fermés, sont formés par le corps de refroidissement (6) de la première unité de thermorégulation (2) conjointement avec le corps de refroidissement (7) de la deuxième unité de thermorégulation (3).

2. Appareil médical de thermorégulation selon la revendication 1, dans lequel le dispositif d'alimentation en fluide présente un dispositif d'amenée de fluide, par lequel le fluide est introduit depuis l'extérieur dans le boîtier (1).

3. Appareil médical de thermorégulation selon la revendication 2, dans lequel le dispositif d'amenée de fluide est prévu pour introduire dans le boîtier (1) au moins une partie du fluide guidé à l'extérieur du boîtier (1) par le dispositif d'évacuation de fluide si bien qu'il résulte un circuit de fluide au moins partiel, dans lequel le fluide introduit dans le boîtier (1) par le dispositif d'amenée de fluide présente une autre température, de préférence plus basse, mieux encore plus élevée et/ou une autre pression, de préférence plus basse, que le fluide guidé à l'extérieur du boîtier (1) par le dispositif d'évacuation de fluide.

4. Appareil médical de thermorégulation selon l'une quelconque des revendications précédentes, dans lequel le dispositif d'alimentation en fluide présente un contenant de fluide disposé dans le boîtier (1).

5. Appareil médical de thermorégulation selon les revendications 2 et 4, dans lequel le dispositif d'amenée de fluide est relié de manière à acheminer du fluide au contenant de fluide.

6. Appareil médical de thermorégulation selon l'une quelconque des revendications précédentes, dans lequel la première unité de thermorégulation (2) et la deuxième unité de thermorégulation (3) présentent une structure sensiblement identique.

7. Appareil médical de thermorégulation selon l'une quelconque des revendications précédentes, dans lequel le corps de refroidissement (6) de la première unité de thermorégulation (2) et le corps de refroidissement (7) de la deuxième unité de thermorégulation (3) présentent respectivement une zone avec des nervures de refroidissement (6a, 7a), et que lesdites zones avec des nervures de refroidissement (6a, 7a) sont tournées l'une vers l'autre.

8. Appareil médical de thermorégulation selon la revendication 7, dans lequel les nervures de refroidissement (6a) du corps de refroidissement (6) de la première unité de refroidissement (2) et/ou les nervures de refroidissement (7a) du corps de refroidissement (7) de la deuxième unité de refroidissement (3) sont fabriquées dans le procédé « Skived-Fin ».

9. Appareil médical de thermorégulation selon l'une quelconque des revendications précédentes, dans lequel la première unité de thermorégulation (2) et la deuxième unité de thermorégulation (3) sont disposées l'une par rapport à l'autre sensiblement en symétrie spéculaire.

10. Appareil médical de thermorégulation selon l'une quelconque des revendications précédentes, dans lequel le dispositif de thermorégulation (2, 3) présente au moins un dispositif de ventilation (14, 15), qui entraîne un flux d'air dans le boîtier (1), qui entre en contact avec la première unité de thermorégulation (2) et la deuxième unité de thermorégulation (3).

11. Appareil médical de thermorégulation selon la revendication 10, dans lequel le flux d'air s'étend de manière sensiblement perpendiculaire par rapport à un sens, dans lequel le corps de refroidissement (6) de la première unité de thermorégulation (2) et le corps de refroidissement (7) de la deuxième unité de thermorégulation (3) sont tournés l'un vers l'autre.

12. Appareil médical de thermorégulation selon la revendication 9 et selon la revendication 10 ou 11, dans lequel le flux d'air s'étend de manière sensiblement parallèle par rapport à un plan de symétrie de la première unité de thermorégulation (2) et de la deuxième unité de thermorégulation (3).

13. Appareil médical de thermorégulation selon l'une quelconque des revendications précédentes, dans lequel l'élément échangeur de chaleur (8) de la première unité de thermorégulation (2) et l'élément échangeur de chaleur (9) de la deuxième unité de thermorégulation (3) sont branchés en série par rapport à l'écoulement traversant de fluide.
